# EUROPEAN PATENT SPECIFICATION

(11) **EP 3 263 498 B1**
(45) Date of publication and mention of the grant of the patent: **08.08.2018**
(21) Application number: 17176079.6
(22) Date of filing: 14.06.2017
(51) Int. Cl.: B65H 45/22, B65H 45/04, A61F 13/15

(54) **AN APPARATUS AND A METHOD FOR FOLDING THE WINGS OF ABSORBENT SANITARY ARTICLES**
VORRICHTUNG UND VERFAHREN ZUM FALTEN DER FLÜGEL VON SAUGFÄHIGEN HYGIENEARTIKELN
APPAREIL ET PROCÉDÉ PERMETTANT DE PLIER LES AILES D'ARTICLES HYGIÉNIQUES ABSORBANTS

(30) Priority: 30.06.2016 IT UA20164788
(43) Date of publication of application: 03.01.2018
(73) Proprietor: Fameccanica.Data S.p.A., 65129 Pescara (IT)
(72) Inventor: SABLONE, Gabriele, I-65125 Pescara (IT); GALLUCCI, Antonio, I-65010 Spoltore (Pescara) (IT)
(74) Representative: Marchitelli, Mauro

(56) References cited:
- WO-A2-2007/029195
- US-A1- 2012 015 791
- US-A1- 2014 135 194

## Description

### Field of the invention

The present invention relates to methods and apparatus for producing disposable absorbent sanitary articles.

More specifically, the invention relates to methods and apparatus for producing absorbent sanitary articles comprising an absorbent central body extending in a longitudinal direction and two lateral wings extending from opposite sides of the absorbent central body.

### Description of the prior art

In the state of the art, female sanitary napkins are well-known, comprising an absorbent central body and a pair of wings extending from opposite sides of the absorbent central body. The wings are intended to be folded around the undergarment edges and to be stuck onto the outer side of the undergarment to retain the absorbent central body in the correct position during use.

For example, US-A-4285343 describes an absorbent sanitary article with an absorbent central body and with two transversal wings that extend laterally from opposite parts of the absorbent central body. The wings can be formed as an integral part of the central body or can be formed separately and fixed to the longitudinal edges of the absorbent central body. The joining lines between the absorbent central body and the wings are flexible so that the wings can be folded onto the absorbent central body for packaging. During use, the wings are opened and folded around the edges of the undergarment and stuck to the outer side of the undergarment. The wings are usually provided with a layer of glue on their outer surface, for the adhesive attachment of the wings onto the outer side of the undergarment.

The most widely used methods for producing absorbent sanitary articles of this type are based on technology known in the sector as "*machine direction*"*,* which envisages forming a continuous chain of blanks of products oriented with their longitudinal axis aligned with the direction of movement of the continuous chain. Generally, absorbent sanitary articles of this type are formed with extended wings, coplanar to the absorbent central body. Before being packaged, the wings are folded, for example, on the inner surface of the absorbent central body, i.e. on the surface that is intended to come into contact with the body of the user, during use.

In the "*machine direction*" manufacturing technique, the folding of the wings on the surface, for example, on the inner surface of the absorbent central body, is carried out by means of a stationary folding device that, due to the movement of the absorbent sanitary articles in the machine direction, engages the wings and rotates them by 180°C between the extended position and the folded position against the surface of the absorbent central body. WO2007/02919 describes a folding system for folding the wings that extend laterally from respective central bodies of a plurality of absorbent articles formed of a continuous composite web movable in a longitudinal direction. The stationary folding device comprises a pair of folding elements that engage the respective wings while the absorbent sanitary articles move in the longitudinal direction.

A folding device of the type described in WO2007/029195 requires that the folding lines of the two wings are spaced apart in a transverse direction by a distance equal to or greater than the maximum width of the absorbent central body. In the case where the transversal distance between the folding lines of the wings is less than the maximum width of the absorbent central body, the folding device intended to carry out the folding of the wings would also fold the parts of the absorbent central body located outside the folding lines. In cases where the absorbent central body has a generic hourglass shape with the wings located in the narrower part of the absorbent central body, the known types of folding devices are unable to fold the wings against the inner surface of the absorbent central body without folding the outer parts of the absorbent central body.

### Object and summary of the invention

The present invention aims to provide a method and an apparatus for folding wings of absorbent sanitary articles that allows the wings to be folded even when the distance between the folding lines is less than the maximum width of the absorbent central body.

According to the present invention, this object is achieved by a folding apparatus and method having the characteristics forming the subject of claims 1 and 7.

The claims form an integral part of the disclosure provided here in relation to the invention.

### Brief description of the drawings

The present invention will now be described in detail with reference to the attached drawings, given purely by way of non-limiting example, wherein:
- Figure 1 is a schematic side view of a folding device according to the present invention,
- Figure 2 is a perspective view illustrating the part indicated by the arrow II in Figure 1,
- Figures 3 and 4 are plan views according to the arrow III of Figure 1 showing the folding sequence of the wings,
- Figures 5, 6, 7, 8 and 9 are cross-sections according to the lines V-V, VI-VI, VII-VII, VIII-VIII and IX-IX, respectively, of Figures 3 and 4, and
- Figure 10 is a cross-section along the line X-X of Figure 6,

### Detailed description

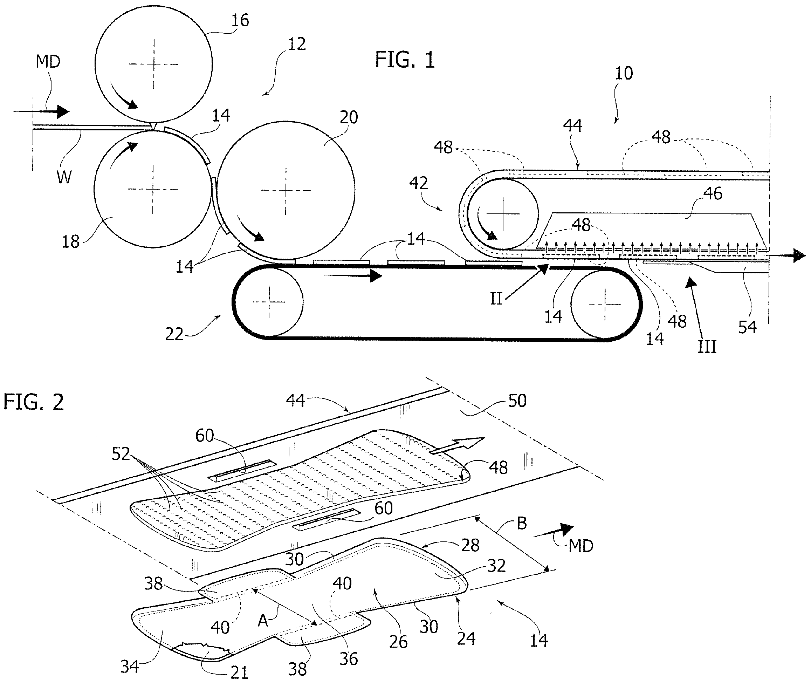

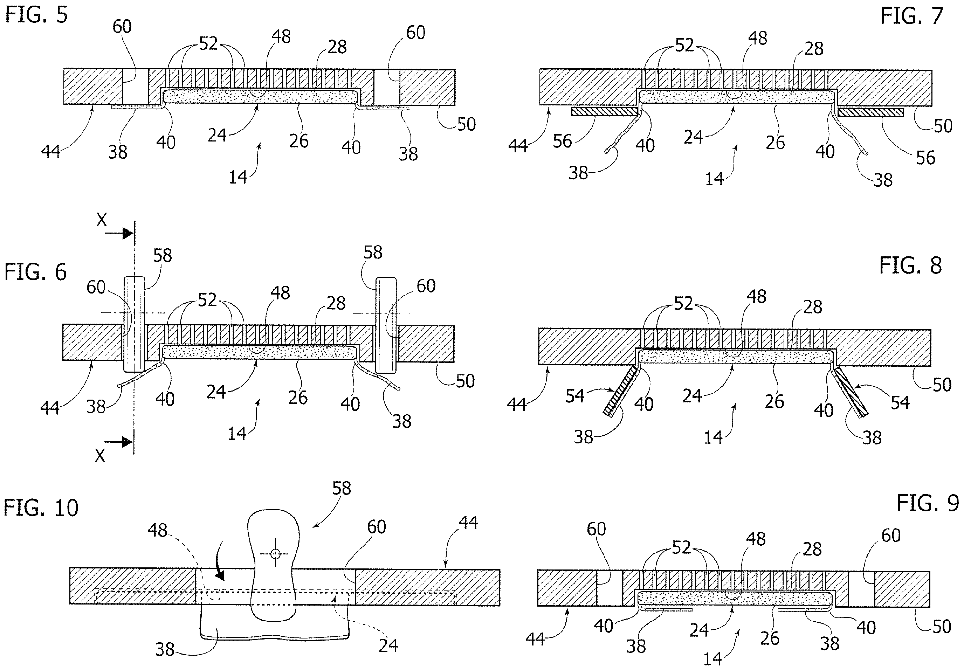

With reference to Figure 1, 10 indicates a folding device for folding wings of disposable absorbent sanitary articles. The folding device 10 is arranged downstream of a cutting unit 12, which performs the cutting of a continuous composite web W in the machine direction MD. Following cutting, the continuous composite web W is subdivided into a continuous succession of absorbent sanitary articles 14, separated from each other. The cutting unit 12 comprises, in a conventional manner, a knives roller 16 and an anvil roller 18. After the cutting, the absorbent sanitary articles 14 can be retained by suction on the periphery of the anvil roller 18. Then, a detaching device 20, typically a suction roller, picks up the absorbent sanitary articles 14 from the cutting unit 12 and places them on a transfer belt 22, which transfers the absorbent sanitary articles to the folding device 10.

With reference to Figure 2, each absorbent sanitary article 14 comprises an absorbent central body 24 having an upper surface 26 intended, during use, to come into contact with the body, and a lower surface 28 intended, during use, to come into contact with an undergarment. The absorbent central body 24 comprises an absorbent core 21 enclosed between a topsheet permeable to body fluids and a backsheet impermeable to body fluids. The absorbent central body 24 has two lateral sides 30 with an arcuate shape, which give the absorbent central body 24 a general hourglass shape, with two end portions 32, 34 wider than a central portion 36.

The absorbent sanitary article 14 comprises a pair of wings 38 located on opposite sides of the central absorbing body 24 and extending laterally outwards from the central portion 36 of the absorbent central body 24. The wings 38 are foldable relative to the central body 24 along respective folding lines 40. The shape of the absorbent central body 24 is such that the distance A between the folding lines 40 - in an orthogonal direction to the direction of movement MD - is less than the maximum width B of the absorbent central body 24.

With reference to Figure 1, the folding apparatus 10 comprises a vacuum conveyor 42 comprising a perforated conveyor belt 44 having a straight portion facing a suction box 46.

With reference to Figure 2, the conveyor belt 44 has a plurality of recessed seats 48 configured for receiving respective absorbent central bodies 24 of absorbent sanitary articles 14. The recessed seats 48 have side walls with a shape corresponding to the side edges 30 of the absorbent central bodies 24, so that the wings 38 of the absorbent sanitary articles 14 extend outwardly from the recessed seats 48 and rest on the outer surface 50 of the conveyor belt 44. The recessed seats 48 are provided with holes 52 that cause the recessed seats 48 to communicate with the suction box 46 so that the absorbent central bodies 24 can be retained by suction in the respective recessed seats 48. The wings 38 can also be retained by suction in an extended position on the outer surface 50 of the conveyor belt 44. The depth of the recessed seats 48 is equal to or greater than the thickness of the absorbent central bodies 24, so that the upper surface 26 of the absorbent central bodies 24 is level with or in a lower position relative to the outer surface 50 of the conveyor belt 44.

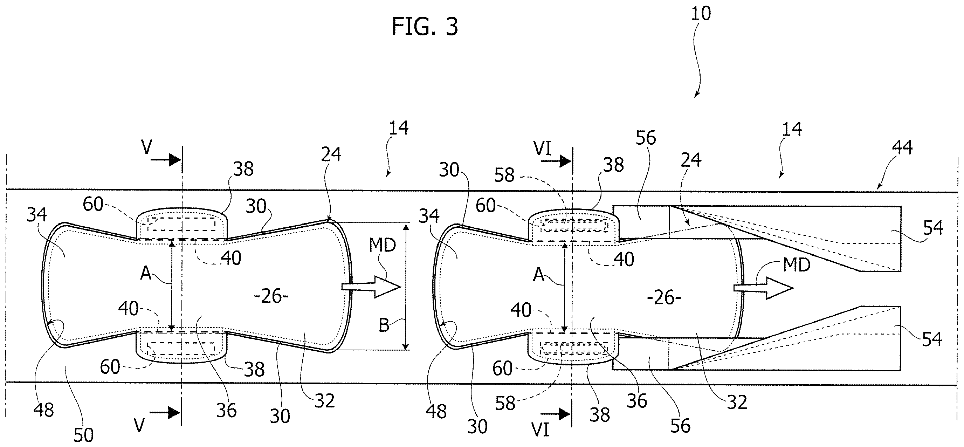

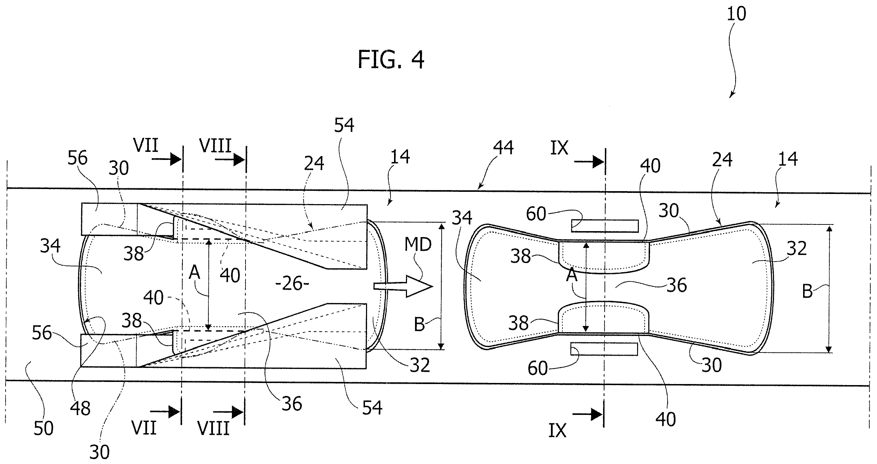

With reference to Figures 3 and 4, the folding apparatus comprises a pair of stationary folding elements 54 facing a straight portion of the conveyor belt 44. The folding elements 54 have respective ends 56 which are inserted between respective wings 38 and the outer surface 50 of the conveyor belt 44. The folding elements 54 have respective helical surfaces that, due to the movement of absorbent sanitary articles 14 in the longitudinal direction MD, fold the wings 38 of the absorbent sanitary articles 14 along the respective folding lines 40. The folding elements 54 are configured to carry out a rotation of 180°C of the wings 38 around the respective folding lines 40, between a position in which the wings 38 extend outwardly to a position in which, in the illustrated example, the wings 38 are folded inside the respective absorbent central body 24 in contact with the respective upper surface 26.

With reference to Figures 6 and 10, the folding apparatus 10 can be provided with detaching means, which detach the wings 38 from the outer surface 50 of the conveyor belt 44 in the vicinity of the ends 56 of the folding elements 54. In the illustrated example, these detaching means comprise a pair of cams 58, rotatable about a transverse axis in phase with the conveyor belt 44, these cams can also be wheels with an eccentric rotational axis. The conveyor belt 44 is provided with through-slots 60 associated with each recessed seat 48, and located at the areas where the wings 38 are provided. The wheels 58 are inserted within respective through-slots 60 when the corresponding recessed seat 48 is located at the end 56 of the folding elements 54. The wheels 58 detach the wings 38 from the outer surface 50 of the conveyor belt 44 and facilitate the insertion of the ends 56 of the folding elements 54 between the outer surface 50 of the conveyor belt 54 and the wings 38, as shown in the right part of Figure 3 and Figure 7.

In the case in which the wings 38 protrude outwardly beyond the outer edges of the absorbent central body 24, the detaching means can be replaced by recessed grooves formed in the outer surface 50 of the conveyor belt 40, which allow the insertion of extension elements of the ends 56 of the folding elements 54 below the wings 38.

As can be seen in Figure 7, the ends 56 of the folding elements 54 engage the wings 38 without interfering with the absorbent central bodies 24 of the absorbent sanitary articles 14. In fact, the wings 38, instead of being coplanar to their respective absorbent central bodies 24, by positioning the absorbent central bodies 24 in the respective lowered seats 48, they are spaced apart from the absorbent central bodies 24 in a direction orthogonal to the outer surface 50 of the conveyor belt 44. This arrangement allows the folding elements 54 to engage the wings 38 without interfering with the parts of the absorbent central bodies 24 located outside the folding lines 40.

Of course, without prejudice to the principle of the invention, the details of construction and the embodiments can be widely varied with respect to those described and illustrated, without thereby departing from the scope of the invention as defined by the claims that follow.

## Claims

1. A folding apparatus for folding absorbent sanitary articles (14) having an absorbent central body (24) and two wings (38) projecting from opposite side edges (30) of said absorbent central body (24), comprising:
- a vacuum conveyor (42) comprising a conveyor belt (44), which holds the absorbent central bodies (24) of said absorbent sanitary articles (14) and moves said absorbent sanitary articles in a longitudinal direction (MD), and
- a pair of stationary folding elements (54) that engage respective wings (38) of said absorbent sanitary articles (14) while they are moved in said longitudinal direction (MD) by said vacuum conveyor (42),
**characterized in that** said conveyor belt (44) comprises a plurality of recessed seats (48) which receive the absorbent central bodies (24) of said absorbent sanitary articles (12), said recessed seats (48) having a shape such that said wings (38) extend laterally outwards from said recessed seats (48) on an outer surface (50) of said conveyor belt (44).

2. An apparatus according to claim 1, wherein said recessed seats (48) have a depth equal to or greater than the thickness of said absorbent central bodies (24) .

3. An apparatus according to claim 1, wherein said recessed seats (48) have side walls with a shape corresponding to the side edges (30) of said absorbent central body (24).

4. An apparatus according to claim 1, wherein said recessed seats (48) are provided with holes (52) that cause said recessed seats (48) to communicate with a suction box (46).

5. An apparatus according to claim 1, comprising detaching means (58) to detach said wings (38) from said outer surface (50) of the conveyor belt (44) at the ends (56) of said folding elements (54).

6. An apparatus according to claim 5, wherein said detaching means comprise a pair of cams (58) rotating in phase with said conveyor belt (44) and arranged to be inserted into respective through-slots (60) formed in the conveyor belt (44) at each recessed seat (48).

7. A method for folding absorbent sanitary articles (14) having an absorbent central body (24) and two lateral wings (38) projecting from opposite side edges (30) of said absorbent central body (24), comprising the steps of:
- moving said absorbent sanitary articles (24) in a longitudinal direction (MD) using a vacuum conveyor (42) comprising a conveyor belt (44), and
- engaging the wings (38) of said absorbent sanitary articles (14) by respective stationary folding elements (54) while the sanitary absorbent articles (14) are moved in said longitudinal direction (MD) by said vacuum conveyor (42),
**characterized in that** said absorbent central bodies (24) of said sanitary absorbent articles (24) are received within recessed seats (48) of said conveyor belt (50) and said lateral wings (38) extend laterally outwards from said recessed seats (48) on an outer surface (50) of said conveyor belt (44).

8. A method according to claim 7, wherein said recessed seats (48) have a depth equal to or greater than the thickness of said absorbent central bodies (24) .

9. A method according to claim 7, comprising the step of detaching said wings (38) from said outer surface (50) of said conveyor belt (44) prior to engaging said wings (38) with said folding elements (54) .

## Patentansprüche

1. Faltvorrichtung zum Falten absorbierender Sanitärartikel (14), die einen absorbierenden mittigen Körper (24) und zwei Flügel (38) haben, die von entgegengesetzten Seitenkanten (30) des absorbierenden mittigen Körpers (24) hervorstehen, die aufweist:
- ein Vakuumförderband (42), das ein Förderband (44) aufweist, das die absorbierenden mittigen Körper (24) der absorbierenden Sanitärartikel (14) hält und die absorbierenden Sanitärartikel in einer Längsrichtung (MD) bewegt, und
- ein Paar stationärer Faltelemente (54), die in die jeweiligen Flügel (38) der absorbierenden Sanitärartikel (14) eingreifen, während sie durch das Vakuumförderband (42) in die Längsrichtung (MD) bewegt werden,
**dadurch gekennzeichnet, dass** das Förderband (44) eine Vielzahl von ausgesparten Aufnahmen (48) aufweist, die die absorbierenden mittigen Körper (24) der absorbierenden Sanitärartikel (12) aufnehmen, wobei die ausgesparten Aufnahmen (48) eine solche Form haben, dass die Flügel (38) von den ausgesparten Aufnahmen (48) an einer Außenfläche (50) des Förderbands (44) seitlich nach außen verlaufen.

2. Vorrichtung nach Anspruch 1, wobei die ausgesparten Aufnahmen (48) eine Tiefe haben, die gleich oder größer als die Dicke der absorbierenden mittigen Körper (24) ist.

3. Vorrichtung nach Anspruch 1, wobei die ausgesparten Aufnahmen (48) Seitenwände in einer Form haben, die den Seitenkanten (30) des absorbierenden mittigen Körpers (24) entspricht.

4. Vorrichtung nach Anspruch 1, wobei die ausgesparten Aufnahmen (48) mit Öffnungen (52) vorgesehen sind, die bewirken, dass die ausgesparten Aufnahmen (48) mit einer Ansaugbox (46) in Austausch stehen.

5. Vorrichtung nach Anspruch 1, die Lösemittel (58) zum Lösen der Flügel (38) von der Außenfläche (50) des Förderbands (44) an den Enden (56) der Faltelemente (54) aufweist.

6. Vorrichtung nach Anspruch 5, wobei die Lösemittel ein Paar von Nocken (58) aufweisen, die sich gleichlaufend mit dem Förderband (44) drehen und angeordnet sind, um in jeweilige durchgehende Schlitze (60) eingefügt zu werden, die im Förderband (44) an jeder ausgesparten Aufnahme (48) ausgebildet sind.

7. Verfahren zum Falten absorbierender Sanitärartikel (14), die einen absorbierenden mittigen Körper (24) und zwei Seitenflügel (38) haben, die von entgegengesetzten Seitenkanten (30) des absorbierenden mittigen Körpers (24) hervorstehen, das die folgenden Schritte aufweist:
- Bewegen der absorbierenden Sanitärartikel (24) in einer Längsrichtung (MD) unter Verwendung eines Vakuumförderbands (42), das ein Förderband (44) aufweist, und
- Eingreifen in die Flügel (38) der absorbierenden Sanitärartikel (14) durch jeweilige stationäre Faltelemente (54), während die absorbierenden Sanitärartikel (14) in der Längsrichtung (MD) durch das Vakuumförderband (42) bewegt werden,
**dadurch gekennzeichnet, dass** die absorbierenden mittigen Körper (24) der absorbierenden Sanitärartikel (24) in den ausgesparten Aufnahmen (48) des Förderbands (50) aufgenommen werden und die seitlichen Flügel (38) von den ausgesparten Aufnahmen (48) an einer Außenfläche (50) des Förderbands (44) seitlich nach außen verlaufen.

8. Verfahren nach Anspruch 7, wobei die ausgesparten Aufnahmen (48) eine Tiefe haben, die gleich oder größer als die Dicke der absorbierenden mittigen Körper (24) ist.

9. Verfahren nach Anspruch 7, das den Schritt des Lösens der Flügel (38) von der Außenfläche (50) des Förderbands (44) aufweist, bevor die Flügel (38) in die Faltelemente (54) eingreifen.

## Revendications

1. Appareil de pliage pour plier des articles hygiéniques absorbants (14) ayant un corps central absorbant (24) et deux ailes (38) faisant saillie à partir de bords latéraux opposés (30) dudit corps central absorbant (24), comprenant :
- un transporteur à vide (42) comprenant une bande transporteuse (44), qui maintient les corps centraux absorbants (24) desdits articles hygiéniques absorbants (14) et déplace lesdits articles hygiéniques absorbants dans une direction longitudinale (MD), et
- une paire d'éléments de pliage fixes (54) qui viennent en prise avec des ailes respectives (38) desdits articles hygiéniques absorbants (14) pendant qu'ils sont déplacés dans ladite direction longitudinale (MD) par ledit transporteur à vide (42),
**caractérisé en ce que** ladite bande transporteuse (44) comprend une pluralité de sièges évidés (48) qui reçoivent les corps centraux absorbants (24) desdits articles hygiéniques absorbants (12), lesdits sièges évidés (48) ayant une forme telle que lesdites ailes (38) s'étendent latéralement vers l'extérieur à partir desdits sièges évidés (48) sur une surface externe (50) de ladite bande transporteuse (44).

2. Appareil selon la revendication 1, dans lequel lesdits sièges évidés (48) ont une profondeur supérieure ou égale à l'épaisseur desdits corps centraux absorbants (24).

3. Appareil selon la revendication 1, dans lequel lesdits sièges évidés (48) ont des parois latérales ayant une forme correspondant aux bords latéraux (30) dudit corps central absorbant (24).

4. Appareil selon la revendication 1, dans lequel lesdits sièges évidés (48) sont pourvus de trous (52) qui amènent lesdits sièges évidés (48) à communiquer avec une caisse aspirante (46).

5. Appareil selon la revendication 1, comprenant des moyens de détachement (58) pour détacher lesdites ailes (38) de ladite surface externe (50) de la bande transporteuse (44) au niveau des extrémités (56) desdits éléments de pliage (54).

6. Appareil selon la revendication 5, dans lequel lesdits moyens de détachement comprennent une paire de cames (58) tournant en phase avec ladite bande transporteuse (44) et agencées pour être insérées dans des fentes traversantes respectives (60) formées dans la bande transporteuse (44) au niveau de chaque siège évidé (48).

7. Procédé de pliage d'articles hygiéniques absorbants (14) ayant un corps central absorbant (24) et deux ailes latérales (38) faisant saillie à partir de bords latéraux opposés (30) dudit corps central absorbant (24), comprenant les étapes consistant :
- à déplacer lesdits articles hygiéniques absorbants (24) dans une direction longitudinale (MD) en utilisant un transporteur à vide (42) comprenant une bande transporteuse (44), et
- à mettre en prise les ailes (38) desdits articles hygiéniques absorbants (14) par des éléments de pliage fixes respectifs (54) pendant que les articles hygiéniques absorbants (14) sont déplacés dans ladite direction longitudinale (MD) par ledit transporteur à vide (42),
**caractérisé en ce que** lesdits corps centraux absorbants (24) desdits articles hygiéniques absorbants (24) sont reçus dans des sièges évidés (48) de ladite bande transporteuse (50) et lesdites ailes latérales (38) s'étendent latéralement vers l'extérieur à partir desdits sièges évidés (48) sur une surface externe (50) de ladite bande transporteuse (44).

8. Procédé selon la revendication 7, dans lequel lesdits sièges évidés (48) ont une profondeur supérieure ou égale à l'épaisseur desdits corps centraux absorbants (24).

9. Procédé selon la revendication 7, comprenant l'étape de détachement desdites ailes (38) de ladite surface externe (50) de ladite bande transporteuse (44) avant la mise en prise desdites ailes (38) avec lesdits éléments de pliage (54).
